(19) Europäisches Patentamt
European Patent Office
Office européen des brevets

(11) EP 0 627 009 B1

(12) **EUROPEAN PATENT SPECIFICATION**

(45) Date of publication and mention of the grant of the patent:
**20.11.1996 Bulletin 1996/47**

(21) Application number: **93905810.3**

(22) Date of filing: **11.02.1993**

(51) Int Cl.[6]: **C12P 17/18**, C07D 498/18, A61K 31/445, C12N 1/20 // (C12N1/20, C12R1:55), (C12P17/18, C12R1:55)

(86) International application number: **PCT/US93/01087**

(87) International publication number: **WO 93/16189 (19.08.1993 Gazette 1993/20)**

(54) **MACROCYCLIC LACTONES AND A PRODUCTIVE STRAIN THEREOF**
MAKROCYCLISCHE LACTONE UND EIN STAMM ZU SEINER HERSTELLUNG
LACTONES MACROCYCLIQUES ET UNE DES SOUCHES PRODUCTIVES S'Y RAPPORTANT

(84) Designated Contracting States:
**AT BE CH DE DK ES FR GB GR IE IT LI LU NL PT SE**

(30) Priority: **17.02.1992 JP 29669/92**

(43) Date of publication of application:
**07.12.1994 Bulletin 1994/49**

(73) Proprietor: **PFIZER INC.**
**New York, N.Y. 10017 (US)**

(72) Inventors:
- **NISHIDA, Hiroyuki**
  **Handa-shi, Aichi-ken (JP)**
- **SAKAKIBARA, Tatsuo**
  **Chita-gun, Aichi-ken (JP)**
- **YAMAUCHI, Yuji**
  **Handa-shi, Aichi-ken (JP)**
- **INAGAKI, Taisuke**
  **Handa-shi, Aichi-ken (JP)**
- **KOJIMA, Yasuhiro**
  **Nishio-shi, Aichi-ken (JP)**
- **KOJIMA, Nakao**
  **Meito-ku, Nagoya-shi (JP)**

(74) Representative: **Wood, David John**
**PFIZER LIMITED,**
**Ramsgate Road**
**Sandwich, Kent CT13 9NJ (GB)**

(56) References cited:
**EP-A- 0 184 162**
**WO-A-92/14737**
**US-A- 5 091 389**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 627 009 B1

## Description

This invention relates to novel macrocyclic lactones, the productive strain therefor, a process for their production and their use. Particularly this invention relates to the novel macrocyclic lactones produced by fermentation of the culture which is deposited as FERM BP-3688, FERM BP-3832 or American Type Culture Collection, ATCC 39471. This invention also relates to the immunosuppressive, antimycotic and antitumor compositions, which include the macrocyclic lactones as active ingredients. Further this invention relates to the process for producing the macrocyclic lactones by fermenting the above deposited cultures.

In 1983, the United States Food and Drug Administration licensed cyclosporin, an anti-rejection drug that revolutionized the field of organ transplant surgery. The drug acts by inhibiting the body's immune system from mobilizing its vast arsenal of natural protecting agents to reject the transplant's foreign protein. Although cyclosporin is effective in fighting transplantation rejection, it suffers drawbacks in causing kidney failure, liver damage and ulcers which in many cases can be very severe. Newer, safer drugs exhibiting less side effects are constantly being searched for. Recently, it has been demonstrated that the macrolide immunosuppressive FK-506, FK-520 (European Patent Publication No. 0184162; Japanese Patent KOKAI Sho61-148181) and its related compounds and rapamycin (United States Patent No. 3929992 and No. 3993749) show a potent immunosuppressive activity both in vitro and in vivo. FK506 is known to cause serious side effects such as kidney damage, but nothing is known about serious side effects of rapamycin in humans (R. L. Simmons and S. C. Wang, Transplant. Proc., 23, 2152-2156, 1991). As to rapamycin derivatives, 27-demethoxyrapamycin is the only natural product (J. A. Findlay et al., Can. J. Chem., 60, 2046-2047, 1982), whereas 21 ,22-Dihydrorapamycin; 19,20,21,22-tetra-hydrorapamycin (United States patent No. 5023262); 42-oxorapamycin (United States Patent No. 5023263); acyl derivatives of rapamycin (United States Patent No. 5100883) and aminoacyl derivatives of rapamycin (European Patent No. 467606) are chemical-synthesized products. In addition, after applicant's Japanese priority date, 27-demethylrapamycin (United States Patent No. 5091389) and 16,27-didemethylrapamycin (United States Patent No. 5093338) were published. Generally speaking, it is in many cases difficult to modify selectively a certain functional group of natural products as well as of macrocyclic lactones. For example, demethylation of the 16 or 27 position of rapamycin, especially the latter, is probably not feasible. On the other hand, the present invention supplies such natural macrocyclic lactones which have never been known.

The object of the present invention is to provide a culture which produces novel macrocyclic lactones and to provide novel macrocyclic lactones with an excellent immunosuppressive activity, antimycotic activity and antitumor activity. Also the object of the present invention is to provide a process for isolating substantially pure novel macrocyclic lactones from the fermentation mixture of Streptomyces hygroscopicus (FERM BP-3688), Actinpolanes sp. (FERM BP-3832) or Streptomyces toyocaensis subsp. humicola (ATCC 39471).

The inventors tested the various cultures for the purpose of discovering novel immunosuppressants. As a result, they discovered that the culture N929-89 (FERM BP-3688), which was isolated from a soil sample collected in Yamaguchi Prefecture, Japan, produced novel macrocyclic lactones which had excellent immunosuppressive activity, antimycotic activity and antitumor activity. Additionally novel macrocyclic lactones having immunosuppressive activity, antimycotic activity and antitumor activity are produced when N902-109 (FERM BP-3832) and N969-34 (ATCC 39471) are cultivated in the presence of a P-450 inhibitor such as 2-methyl-1,2-di(3-pyridyl)-1-propanone and rapamycin, respectively.

The macrocyclic lactones of the present invention were produced by aerobic fermentation of N929-89, N902-109 or N469-34 in a nutrient medium. There are at least six highly related novel macrocyclic lactones produced by N929-89. They are described herein as N929-89A, N929-89B, N929-89$C_a$, N929-89$C_b$, N929-89$C_g$ and N929-89M, which have excellent immunosuppressive activity, antimycotic activity and an antitumor activity. At least fifteen compounds are produced by N902-109. They are described herein as N902-109A, N902-109$B_1$, N902-109$B_2$, N902-109C, N902-109D, N902-109$E_1$, N902-109$E_2$, N902-109F, N902-109G, N902-109H, N902-1091, N902-109J, N902-109K, N902-109L and N902-109M. Similarly at least six macrocyclic lactones are produced by N469-34. They are described herein as N469-34A, N469-34B, N469-34C, N469-34D, N469-34E and N469-34F.

These compounds are obtained by the fermentation of N929-89, N902-109 or N469-34 and are recovered in substantially pure form.

The culture N902-109 is described in an application named "Novel rapamycin producing culture " to the Japanese Patent Office (Application No. Hei 4-107612) by the same applicants as this patent application and its taxonomical properties are written in that application. In addition, the taxonomical properties of N469-34 are described in the United States Patent No. 4543334.

On the other hand, N929-89 is first proposed by this invention. The culture N929-89 was planted from a slant into ATCC #172 broth and grown for four days at 28°C on a shaker. It was then centrifuged for 20 minutes, washed three times with sterile water and planted on media commonly used for identification of members of the Actinomycetales. The culture was incubated at 28°C and the results were read at varying times but most commonly were taken at 14 days. The colors were described in common terminology, but exact colors were determined by comparisons with color

chips from the Color Harmony Manual, fourth edition. The methods of whole-cell amino acid and sugar analyses are those described in Becker, B. et al., Appl. Microbiol., 12, 421423, 1964; and in Lechevalier, M. P., J. Lab. Cin. Med., 71, 934-944, 1968. Identification media used for the characterization of the culture are as follows:

1. Tryptone-Yeast Extract Broth (ISP No. 1 medium, Difco).
2. Yeast Extract-Malt Extract Agar (ISP No. 2 medium, Difco).
3. Oatmeal Agar - (ISP No. 3 medium, Difco).
4. Inorganic Salts-Starch Agar (ISP No. 4 medium, Difco).
5. Glycerol-Asparagine Agar (ISP No. 5 medium, Difco).
6. Peptone-Yeast Extract Iron Agar (ISP No. 6 medium, Difco).
7. Czapek-Sucrose Agar (S. A. Waksman, The Actinomycetes, Vol. 2, medium no. 1, p. 328, 1961).
8. Glucose-Asparagine Agar (Ibid, medium no. 2, p. 328).
9. Bennett's Agar (Ibid, medium no. 30, p. 331)
10. Emerson's Agar (Ibid, medium no. 28, p. 33
11. Nutrient Agar (Ibid, medium no. 14, p. 330).
12. Gordon and Smith's Tyrosine Agar (R. E. Gordon and M. M. Smith, J. Bacterial., 69, 147-150, 1955).
13. Casein Agar (Ibid).
14. Calcium Malate Agar (S. A. Waksman, Bacteriol. Rev., 21, 1-29, 1957).
15. Gelatin (R. E. Gordon and J. M. Mihm, J. Bacterial. 73, 15-27, 1957).
16. Starch (Ibid).
17. Organic Nitrate Broth (Ibid).
18. Dextrose Nitrate Broth (S. A. Waksman, The Actinomycetes, Vol. 2, medium no. 1, p. 328, 1961, with 3 g dextrose substituted for 30 g sucrose and agar omitted).
19. Potato Carrot Agar (M. P. Lechevalier, J. Lab. and Clinical Med., 71, 934-944, 1968, but use only 30 g potatoes, 2.5 g carrots and 20 g agar).
20. 2% Tap Water Agar.
21. Skim Milk (Difco).
22. Cellulose utilization (H. L. Jensen, Proc. Linn. Soc., N.S.W. 55, 231-248, 1930. M. Levine and H. W. Schoenlein, A Compilation of Culture Media, medium no. 2511, 1930).
23. Carbohydrate Utilization (ISP No. 9 medium, Difco).
24. Temperature Range (ISP No. 2 plus 50 ml of coconut milk per liter of the medium).

Yeast Extract-Malt Extract Agar - Growth good, pale gray to gray (near gray series 3 cb, 3dc, 3 fe), moderately raised, smooth to wrinkled, aerial mycelium same as surface; reverse yellowish brown, brown, grayish to black (3 gc, 3 ie, 3 le, near gray series 3 fe, 3 ml); soluble pigment pale yellowish brown (3 gc).
Oatmeal Agar - Growth good, gray (near gray series 3 dc, 3 fe, 5 fe), moderately raised, smooth to granular, aerial mycelium same as surface; reverse gray (near gray series 3 fe); soluble pigment pale yellowish (1 1/2 ca).
Inorganic Salts-Starch Agar - Growth good; white, pale gray to gray (near gray series 3 cb, 3 bc, 3 fe), raised, smooth to slightly wrinkled, aerial mycelium same as surface; reverse pale gray to gray (near gray series 3 dc, 3 fe); no soluble pigment.
Glycerol-Asparadine Agar - Growth poor to moderate, cream with some white to pale gray dots (near gray series 3 cb, 3 dc), slightly raised, smooth to granular, aerial mycelium white to pale gray (near gray series 3 cb, 3 dc); reverse cream with some pale gray to gray dots (near gray series 3 dc, 3 fe); soluble pigment cream (2 ca).
Czapek-Sucrose Agar - Growth moderate to good, cream to pale gray (2 ca, near gray series 3 cb), slightly raised, smooth to granular, aerial mycelium pale gray (near gray series 3 cb, 3 bc); reverse colorless to pale gray (near gray series 3 bc); no soluble pigment.
Glucose-Asparadine Agar - Growth moderate to good, yellowish (1 1/2 ea, 1 1/2 ga, 2 ea), slightly raised, smooth to granular, aerial mycelium white to pale gray (near gray series 3 cb); reverse yellowish (2 ga, 2 ea); soluble pigment yellowish (2 ga).
Gordon and Smith's Tyrosine Agar - Growth moderate, tan (near 3 gc), thin to slightly raised, smooth, aerial mycelium white to pale gray (near gray series 3 cb); reverse tan (3 gc); soluble pigment yellowish brown (3 nc).
Casein Agar - Growth good, white with some gray dots (near gray series 3 dc, 3 fe), moderately raised, smooth to slightly granular, aerial mycelium white, pale gray to gray (near gray series 3 dc, 3 fe); reverse yellowish (2 ea); soluble pigment dark yellowish (2 Pe).
Bennett's Agar - Growth good; white, pale gray to gray (near gray series 3 dc, 3 fe); raised, smooth but slightly wrinkled at end of streak, aerial mycelium same as surface; reverse white, pale gray to gray (near gray series 3 dc, 3 fe); soluble pigment pale yellowish (2 ea).
Emerson's Agar - Growth moderate to good, white to pale gray (near gray series 3 cb), moderately raised, smooth to

wrinkled, aerial mycelium same as surface; reverse yellowish (2 ea); soluble pigment yellowish brown (3 nc).

Nutrient Agar - Growth poor to moderate, pale yellowish (between 2 ca and 2 ea), thin to slightly raised, smooth, no aerial mycelium; reverse cream (2 ca); no soluble pigment.

Calcium Malate Agar - No growth to a few small cream dots (2 ca), no aerial mycelium; reverse cream; no soluble pigment.

Gelatin Agar - Growth moderate, white to dark yellowish (2 gc), moderately raised, smooth to granular or slightly wrinkled toward the edge, aerial mycelium white; reverse cream to yellow (2 ca, 2 ea); no soluble pigment.

Starch Agar - Growth moderate, white to dark yellow (2 gc), moderately raised, smooth to granular or wrinkled toward the edge, aerial mycelium white; reverse cream to yellow (2 ca, 2 ea); no soluble pigment.

Potato Carrot Agar - Growth moderate, pale gray to gray (near gray series 3 cb, 3 dc, 5 fe), slightly to moderately raised, smooth to granular, aerial mycelium same as surface; reverse gray (near gray series 5 fe); no soluble pigment.

Tap Water Agar - Growth poor to moderate, gray (near gray series 3 fe, 5 fe), thin to slightly raised, smooth, aerial mycelium gray (near gray series 3 fe, 5 fe); reverse gray (near gray series 5 fe, 5 ih); no soluble pigment.

The morphological observations were made on oatmeal agar after 14 days of incubation: spore mass in Gray color-series; spore chains in Section Spirales, tightly coiled or coiled into an irregular mass, occasionally hooked or slightly open, with up to 7 turns, of small diameter (3 - 4 mm); 10 to 30 spores per spore chain, often aggregated into big masses; spore masses forming black hygroscopic patches at 24 days of incubation; sporophores monopodially branched, occasionally verticillately branched; Spores oval, elliptical, short-rod to rod-shaped, often with both ends not parallel to each others, 1 - 1.6 (-1.8) x 0.8 - 1.1 (-1.2) mm; spore surface rugose, as revealed by scanning electron microscopy.

Melanin not produced; hydrogen sulfide produced; gelatin liquefied; starch hydrolyzed; nitrate reduced to nitrite; poor growth and no decomposition on both cellulose broths; coagulation and peptonization on milk; casein digestion positive; tyrosine digestion negative. Carbohydrate utilization: glucose, arabinose, fructose, inositol, mannitol, raffinose, sucrose and xylose utilized; rhamnose not utilized.

The culture showed moderate to good growth at 21°C; good to excellent growth at 28°C; moderate growth at 37°C; and no growth at 45°C.

The whole-cell hydrolysates contained LL-diaminopimelic acid, glucose, mannose and ribose.

Summarizing the above mycological character, the culture N929-89 is characterized by the gray spores in mass, the negative melanin reaction, and the rugose spores which are arranged in spirally coiled chains. After 24 days of incubation, some hygroscopic patches of spore chains were produced on yeast extract-malt extract agar, oatmeal agar, inorganic salts-starch agar, and potato carrot agar. The colors of the substrate mycelium ranged from cream, yellow, pale gray to gray. Except for some tints of cream, pale yellow, yellow and yellow-brown, there were no distinct soluble pigments produced. The culture utilized glucose, arabinose, fructose, inositol, mannitol, raffinose, sucrose and xylose, but did not utilize rhamnose. The whole-cell hydrolysates indicate the presence of LL-diaminopimelic acid and the absence of diagnostic sugars.

On the basis of the above properties, the culture N929-89 is most similar to the properties of Streptomyces hygroscopicus in Streptomyces Species. Except for positive utilization of sucrose and raffinose and failure to utilize rhamnose, culture N929-89 fits into the description of the neotype strain of S. hygroscopicus published in Int. J. Syst. Bacterial. 22, 265-394, 1972. According to Tresner and Backus who proposed a broadened concept of this species published in Appl. Microbiol. 4, 243-250, 1956, the utilization of carbon sources differs among strains. Thus, culture N929-89 is considered as a new strain of Streptomyces hygroscopicus (Jensen) Waksman and Henrici. The culture N929-89 has been deposited at the Fermentation Research Institute, Agency of Industrial Science and Technology, Japan, assigned as Streptomyces hygroscopicus F. D. 29009 with an accession number FERM BP-3688 under the Budapest Treaty on December 19, 1991.

When the culture N929-89 is compared with the rapamycin-producing culture Streptomyces hygroscopicus ATCC 29253 described by Vezina et al. in J. Antibiotics; 28, 721-726, 1975, it differs in the production of hydrogen sulfide, the positive utilization of sucrose and the failure to utilize rhamnose. In addition, on glycerol-asparagine agar and Bennett's agar the colony reverse of culture N929-89 is cream and pale gray to gray rather than pale yellowish to pinkish and pale yellow to greenish, respectively. On glycerol-asparagine agar the soluble pigment of culture N929-89 is cream whereas that of culture ATCC 29253 is pale pink.

The following are preferable for fermentation of the culture. In small scale fermentations, after corking a flask with a cotton stopper, the spore or the nurse cell of Streptomyces hygroscopicus FERM BP-3688 is inoculated in sterilized medium. The flask was shaken at 28 ° C for 3 to 10 days. Larger scale fermentations may be carried out by preparing shake tanks with agitator, aerating apparatus and sterilized apparatus. The spore or the nurse cell of Streptomyces hygroscopicus FERM BP-3688 is inoculated in sterilized medium in a tank. The PH of the medium is 5 to 9, preferably 6 to 7.5. The fermentation is carried out at a temperature from about 20°C to about 40°C under submerged conditions with agitation and/or aeration in a medium. The degree of aeration may be changed by some factor such as size of the fermenter and agitation speed. In general the agitation is carried out from 0 to 2,000 rpm more preferably from 100

to 360 rpm and aeration is carried out from 0 to 500%, more preferably from 80 to 120% volume of medium.

The culture N469-34 in this invention can be fermented in a similar manner to above mentioned N929-89 except for adding rapamycin to the fermentation mixture and the culture N902-109 can be also fermented in a similar manner to N929-89 with exception that 2-methyl-1,2-di(3-pyridyl)-1-propanone, which is an inhibitor of metabolic enzyme P-450, was added to the fermentation broth.

The novel compounds of this invention can be obtained from the fermentation broth by cultivating Streptomyces hygroscopicus FERM BP-3688. The novel macrocyclic lactones of the present invention can be isolated by extraction and various chromatographic techniques.

The existence of the macrocyclic lactones can be confirmed by measuring a human mixed lymphocyte reaction (MLR) and the various kinds of spectra (e.g., UV spectra show the inherent spectra of the macrocyclic lactones). Thus at least six macrocyclic lactones, N929-89A, N929-89B, N929-89Ca, N929-89Cb, N929-89Cg and N929-89M are isolated by the fermentation of the culture N929-89. At least fifteen macrocyclic lactones, N902-109A, N902-109$B_1$, N902-1098$_2$, N902-109C, N902-109D, N902-109$E_1$, N902-109$E_2$, N902-109F, N902-109G, N902-109H, N902-1091, N902-109J, N902-109K, N902-109L and N902-109M are isolated by the fermentation of the culture N902-109. In addition, at least six macrocyclic lactones, N469-34A, N469-34B, N469-34C, N469-34D, N469-34E and N469-34F are isolated by the fermentation of the culture N469-34.

The physicochemical properties of the macrocyclic lactones whose structures were determined are shown in the following scheme.

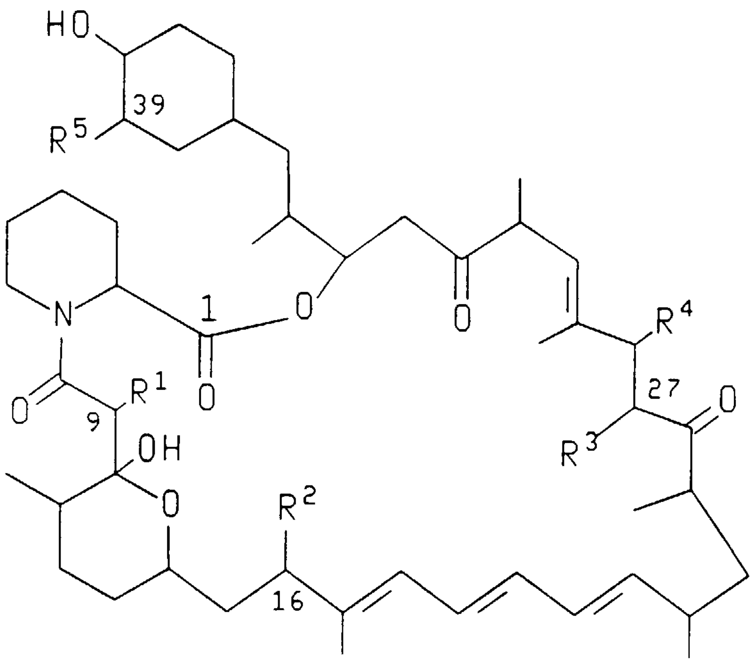

In the planar structures, the substituents of the macrocyclic lactones are summerized in Table 1.

Table 1

| Compounds | $R^1$ | $R^2$ | $R^3$ | $R^4$ | $R^5$ |
|---|---|---|---|---|---|
| N929-89A | O | OH | OH | OH | $OCH_3$ |
| N929-89B | O | OH | $OCH_3$ | OH | $OCH_3$ |
| N929-89Ca | O | $OCH_3$ | H | OH | OH |
| N929-89Cb | O | $OCH_3$ | OH | OH | $OCH_3$ |
| N902-109A | O | $OCH_3$ | $OCH_3$ | H | $OCH_3$ |
| N902-109$B_1$ | $H_2$ | $OCH_3$ | $OCH_3$ | OH | $OCH_3$ |
| N902-109C | $H_2$ | $OCH_3$ | H | OH | $OCH_3$ |
| N902-109H | $H_2$ | $OCH_3$ | H | OH | OH |
| N469-34A | O | $OCH_3$ | $OCH_3$ | OH | OH |

The macrocyclic lactones which are isolated in this invention are described as the plane chemical structures in this specification. As the macrocyclic lactones possess at least 11 asymmetric carbon atoms, they are capable of occurring in various stereoisomeric forms or configurations. Hence, when those skilled in the art use the compounds of this invention, they may choose any desired isomers such as optical isomers and diastereomers from among the objective compounds of the present invention according to their application purpose.

The measurement of the human MLR activity of the macrocyclic lactones of this invention carried out by standard procedures which are described in the literature (D. P. Dubey et al., in Manual of Clinical Laboratory Immunology, 3rd Ed., pp847-858, 1986). The results are summarized in Table 2. The cytotoxicities of the macrocyclic lactones were measured by the standard procedure (T. Mosmann, J., J. Immunol. Methods, 65, 55-63, 1983). The concentration of their cytotoxicities is several ten times higher than that of their MLR activities. The facts show that these novel macrocyclic lactones have an excellent immunosuppressive activity.

Table 2

| Compounds | MLR activity($IC_{50}$, mg/ml) |
|---|---|
| N929-89A | 8 |
| N929-89B | 2.8 |
| N929-89$C_a$ | 47 |
| N929-89$C_b$ | 2.8 |
| N929-89$C_g$ | 42 |
| N929-89M | 0.1 |
| N902-109A | >300 |
| N902-109$B_1$ | 82 |
| N902-109$B_2$ | 12 |
| N902-109C | 5 |
| N902-109D | 8 |
| N902-109E | 61 |

Table 2 (Con't)

| Compounds | MLR activity($IC_{50}$, mg/ml) |
|---|---|
| N902-109F | 200 |
| N902-109H | 32 |
| N902-109J | 280 |
| N469-34A | 12 |
| Rapamycin | 10 |

The activities against <u>Candida albicans</u> of compounds of the present invention were determined by an agar plate dilution method and were showed by the diameter of inhibition zone. The paper disk (8 mm, Advantic) and agar medium for neomycin assay (Difco) were used in the tests. The results are summarized in Table 3. As the novel macrocyclic lactones inhibit the growth of <u>Candida albicans,</u> they have an antifungal activity.

Table 3

| Compounds | Concentration (mg/disk) | The diameter of inhibition zone (mm) |
|---|---|---|
| N929-89A | 40 | 12 |
| N929-89B | 40 | 25 |
| N929-89$C_a$ | 40 | 15 |
| N929-89$C_b$ | 40 | 15 |
| N929-89$C_g$ | 40 | 12 |
| N929-89M | 40 | 12 |
| Rapamycin | 40 | 25 |

All novel macrocyclic lactones of the present invention show an immunosuppressive activity, an antimycotic activity and an antitumor activity. As immunosuppressives, the macrocyclic lactones of this invention can be used in the same manner as rapamycin. The preferable compounds are N902-109C, N902-109D and N469-34A.

The novel macrocyclic lactones of this invention can be administered to a mammalian subject, for example a human subject, as an immunosuppressive agent either alone, or in combination with a inert diluent in a pharmaceutical composition. In many cases, the compounds can be applied by parenteral or oral administration in the form of compositions which contain the macrocyclic lactones as active ingredients and pharmaceutically acceptable carriers or excipients. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations. In addition, if needed, auxiliary, stabilizing and coloring agents and perfumes may be used.

While the dosage of an immunosuppressive effective amount of the macrocyclic lactones depends upon each individual human patient to be treated, a daily dose of about 1.0 to about 1000 mg, can be given. For treating transplantation rejection, daily doses of 5 mg, 10 mg, 50 mg, 100 mg, 250 mg or 500 mg are generally preferred. The same dosages can be given when the compounds are used as antimycotic or antitumor agents.

The present invention is illustrated by the following examples. However, it should be understood that the invention is not limited to the specific details of these examples. UV spectra were recorded in methanol on a JASCO Ubest-30 UV/VIS spectrophotometer. All NMR spectra were measured in $CDCl_3$ by a Bruker NMR spectrometer (AM-500) unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad. LSI (Liquid Secondary Ion) mass spectra were measured by Kratos mass spectrometer(model 1S) using NaI-matrix of dithiothreitol : Dithioerythritol (3:1).

Example 1

One hundred ml of Medium-1 (glucose 2%, polypeptone 0.5%, beef extract 0.3%, wheat gluten 0.5%, yeast extract 0.5%, blood meal 0.3% and $CaCO_3$ 0.4%, PH 7.0-7.2) in a 500 ml flask was inoculated with a vegitative cell suspension from a slant culture of Streptomyces hygroscopicus FERM BP-3688. The flask was shaken at 28°C for 4 days on a rotary shaker with 7-cm throw at 220 rpm.

Five shake flasks containing Medium 1 (150ml) were inoculated with 7.5ml of the grown culture. These flasks were shaken at 28°C for 4 days on the rotary shaker, which is called the second seed culture.

The second seed culture in the 5 shake flasks were used to inoculate four 6-liter (L) fermentation vessels containing 3 L of sterile medium (Medium-2: glucose 1 %, corn starch 2%, NZ amine type A 0.5%, wheat embryo 0.5%, yeast extract 0.5%, $CoCl_2.6H_2O$ 0.0001%, $CaCO_3$ 0.4%, PH 7.2-7.4). Aeration was carried out at 28°C for 3 days with 1,700 rpm at 3 L per minute.

The fermentation broth (15 L) was freeze-dried and extracted three times with 5 L of 15 L of ethyl acetate. The extract was dried over anhydrous $Na_2SO_4$ and evaporated to afford the oily residue (10.4 g) which includes the macrocyclic lactones. The oily residue (10 g) was loaded on a silica gel column (250 g) eluting with the step gradiented solvent of n-hexane (1,000 ml)/ ethyl acetate : n-hexane = 1:1(1,000 ml)/n-hexane : ethyl acetate = 1:2 (1,500 ml)/ ethyl acetate (1,000 ml)/ and ethyl acetate : acetone = 2:1 (750 ml). Fraction showing the activity was applied to a Sephadex (registered trade mark) LH-20 column and eluted with methanol. Fractions containing the macrocyclic lactones was further applied to a Chemcosorb (registered trade mark) 5ODS-UH column (20 x 250 mm) eluting with methanol : water = 4:1 at flow rate of 5 ml/min. Detection was made by UV absorbance at 305 nm. The eluted peaks were collected to yield the compound N929-89A (6.4 mg), compound B (1.0 mg), compound N929-89$C_a$ (1.0 mg),

compound N929-89$C_b$ (1.0 mg), compound N929-89$C_g$ (2.1 mg) and compound N929-89M (0.5 mg), which show the Candida albicans activity and MLR activity. The six compounds were separated by HPLC using the Chemcosorb (registered trade mark) 5ODS-UH (4.6 mm x 150 mm) eluting with methanol : water = 7:3 to 10:0 at flow rate of 0.7 ml/min at 40°C. The retention times of the compounds are shown below. The detection was carried out by UV at 280 nm.

Table 4

| Compound | Retention time (min) |
|---|---|
| N929-89A | 6.58 |
| N929-89B | 7.35 |
| N929-89$C_a$ | 11.31 |
| N929-89$C_b$ | 11.46 |
| N929-89$C_g$ | 11.10 |
| N929-89M | 0.29 |

The physicochemical properties of the six macrocyclic lactones obtasined here, N929-89A, N929-89B, N929-89$C_a$, N929-89$C_b$, N929-89$C_g$ and N929-89M are folows.

The existence of N929-89$C_g$ and N929-89M are supported by their LSI mass spectra. In these cases, ion peaks of the compounds N929-89$C_g$ and N929-89M show the 28 and 44 mass units smaller than that of the compound B. The $^1$H NMR spectra of the compounds $C_g$ and M also supported the existence of the macrocyclic lactones.

| | N929-89A | N929-89B |
|---|---|---|
| Appearance | White powder | White powder |
| UVImax (MeOH) | 267, 277, 288 | 267, 277, 288 |
| Molecular weight | 886.10 | 900.17 |
| Molecular formula | $C_{49}H_{75}NO_{13}$ | $C_{50}H_{77}NO_{13}$ |
| LSIMS $[M+Na]^+$ | 908.5$[M+Na]^+$ | 922.5$[M+Na]^+$ |
| $^1H$ NMR $\delta$ (ppm) | 3.41 (3H, s, -OMe) | 3.34 (3H, s, -OMe)<br>3.41 (3H, s, -OMe) |

| N929-89Ca | N929-89Cb | N929-89Cg |
|---|---|---|
| White powder | White powder | White powder |
| 267, 277, 288 | 267, 277, 288 | 267, 277, 288 |
| 870.14 | 900.17 | - |
| $C_{49}H_{75}NO_{12}$ | $C_{50}H_{77}NO_{13}$ | - |
| 892.5$[M+Na]^+$ | 922.5$[M+Na]^+$ | 894.5$[M+Na]^+$ |
| 3.14(3H, s, -OMe) | 3.14 (3H, s, -OMe)<br>3.41 (3H, s, -OMe) | 3.41 (3H, s, -OMe) |

| N929-89M |
|---|
| White powder |
| 267, 277, 288 |
| - |
| - |
| 878.5$[M+Na]^+$ |
| 3.41 (3H, s, -OMe) |

Example 2

A seed culture (5 ml) as described in Example 1 was inoculated into 100 ml of the Medium 2 in a 500 ml shake flask. The flask was shaken at 28°C for 3 days on a rotary shaker with 7 cm throw at 220 rpm. It was apparent that fermentation broth included the macrocyclic lactones, since the fermentation broth (100 ml) which was obtained the above showed the anti-Candida albicans activity and the MLR activity. The compounds N929-89A, N929-89B, N929-89$C_a$, N929-89$C_b$, N929-89$C_g$ and N929-89M are able to be isolated in a similar manner to example 1.

Example 3

A seed culture of Streptomyces hygroscopicus FERM BP-3688 as described in Example 1 was inoculated into 1

L of the Medium 1 in three 3 L shake flask. The second seed culture was prepared by shaking the flask at 28°C for 4 days on a rotary shaker with 7 cm throw at 220 rpm.

The second seed culture (3L) was inoculated into 70 L of the Medium 2 as described in Example 1 in 100 L stainless fermenter. The fermenter was shaken at 28°C for 3 days at 360 rpm.

The whole jar fermentation broth (70 L) was extracted with 70 L of ethyl acetate. The ethyl acetate layer was dried over anhydrous $Na_2SO_4$ and concentrated to dryness. The oily residue contained the macrocyclic lactones and show the anti-Candida albicans activity and the MLR activity. The compounds N929-89A, N929-89B, N929-89$C_a$, N929-89$C_b$, N929-89$C_g$ and N929-89M are able to be isolated in a similar manner to example 1.

Example 4

One hundred ml of Medium-1 in example 1 in a 500 ml flask was inoculated with a vegitative cell suspension from a slant culture of Actinoplanes sp. FERM BP-3832. The flask was shaken at 26°C for 4 days on a rotary shaker with 7-cm throw at 220 rpm, which is called the first seed culture.

Five shake flasks containing Medium 1 (150 ml) were inoculated with 7.5 ml of the first seed culture 1. These flasks were shaken at 26°C for 3 days on the rotary shaker, which is called the second seed culture.

The second seed culture in example 1 in the 5 shake flasks were used to inoculate four 6-liter (L) fermentation vessels containing 3 L of sterile medium. Aeration was carried out at 26°C for 24 hours with 1,700 rpm at 3 L per minute. After that, 2-methyl-1,2-di(3-pyridyl-1-propanone in water was added to it, where the resulting concentration of the compound was 5mM, and the mixture was stirred for 72 hours.

The fermentation broth (30 L) was freeze-dried and extracted twice with 30 L of ethyl acetate. After filtrating the ethyl acetate extract through the supercell, the extract was dried over anhydrous Na2SO4 and evaporated to afford the oily residue (42 g) which includes the macrocyclic lactones. The oily residue was loaded on a silica gel column (200 g) eluting with the step-gradiented solvent of n-hexane: ethyl acate = 2:1 (2,000 ml)/n-hexane : ethyl acetate = 1:1 (2,000 ml). Then n-hexane : ethyl acetate = 2:1 (3,000 ml) and ethyl acetate (3,000 ml). The eluent was collected into 500 ml Fractions (No. 1 to No. 12). Further the column was washed with ethyl acetate: MeOH = 9:1 (2,000ml) to give three fractions of 667ml each. The hexane : ethyl acetate = 1:1 fraction was concentrated and the residue was applied to a Sephadex (registered trade mark) LH-20 column eluting with methanol to give 27 fractions of 8ml each. The concentrated 23rd fraction was separated by HPLC of a Chemcosorb (registered trade mark) 5ODS-UH column (20 x 250 mm) eluting with methanol : water = 85:15 at flow rate of 5 ml/min monitoring with UV absorbance at 305 nm. The five macrocyclic lactones, N902-109$B_2$ (8.1 mg), N902-109D (9.8 mg), N902-109H (0.5 mg), N902-109C (9.1 mg) and N902-109$B_1$ (4.5 mg) were obtained after retention times of 40.4 min, 47.6 min, 63.9 min, 82.6 min and 86.8 min, respectively. In addition, nine peaks, after retention times of 17.7 min, 21.4 min, 23.1 min, 25.1 min, 26.7 min, 28.4 min, 29.6 min, 33.2 min and 69.2 min respectively were observed and were shown to have the same UV spectra as the said above 5 macrocyclic lactones. The above five compounds were separated by HPLC on a Chemcosorb (registered trade mark) 5ODS -UH (4.6 mm x 150 mm) eluting with linear-gradiented solvent, methanol : water = 7:3 to 10:0 for 30 min at flow rate of 0.7 ml/min at 40°C. The retention times of the compounds are shown in Table 6.

Table 6

| Compound | Retention time (min) | $UV\lambda_{max}$ | $LSIMS[M+Na]^+$ |
|---|---|---|---|
| N902-109A | 24.5/25.3 | 267,277,288 | 920.6 |
| N902-109B | 16.2/23.5 | 267,277,288 | 922.5 |
| N902-109$C_a$ | 17.6/23.4 | 267,277,288 | - |
| N902-109$C_b$ | 18.3/23 | 267,277,288 | 892.5 |
| N902-109$C_g$ | 19.2 | 267,277,288 | - |
| N902-109M | 17.0/21.2 | 267,277,288 | 878.5 |

Example 5

One hundred ml of Medium-1 in example 1 in a 500 ml flask was inoculated with a vegitative cell suspension from a slant culture of Streptomyces toyocaensis subsp. fumicola ATCC 39471. The flask was shaken at 26°C for 4 days on a rotary shaker with 7-cm throw at 220 rpm.

Three shake flasks containing Medium 1 (150 ml) were inoculated with 7.5 ml of the grown culture. These flasks were shaken at 26°C for 3 days on the rotary shaker, which is called the second seed culture.

One hundred and fifty ml of the second seed culture were added to inoculate four 6-liter (L) fermentation vessels containing 3 L of sterile medium (Medium-3: glucose 3%, corn starch 1%, corn steep powder 0.75%, sungross 0.5%,

cotton powder 0.5%, $CaCO_3$ 0.4%, $CoCl_2 . 6H_2O$ 0.0001%, PH 7.0). After aeration was carried out at 26°C for one day with 1,700 rpm at 3 L per minute, 30 ml of rapamycin sterile water solution (5 mg/ml) was added to each fermentation broth. Then aeration was continued for an additional 2 days at 26°C.

The fermentation broth (15 L) was freeze-dried and extracted three times with 15 L of ethyl acetate. The extract was dried over anhydrous $Na_2SO_4$ and evaporated to afford the oily residue which includes the macrocyclic lactones. The oily residue was loaded on a silica gel column (100 g) eluating with the step-gradiented solvent of chloroform (1,000 ml)/chloroform : methanol = 100:1 (1, 000 ml)/chloroform:methanol= 100:4 and methanol (1000 ml). The fraction showing the activity was applied to a Sephadex LH-20 (trademark) column and eluted with methanol. The fraction containing the macocyclic lactones was further applied to a Chemcosorb (registered trade mark) 30C18 column 220 ml, 20 x 250 mm) eluting with methanol : water = 4:1. Then the fraction which contains macrocyclic lactones was applied to a cupsell puck SG120 C18 (Shiseido's trademark, 20 mm x 250 mm) eluating with methanol : water = 4:1 at flow rate of 7.5 ml/min. Detection was made by UV absorbance at 310 nm. The eluted peaks were collected to yield the mixture (43.5 mg), which shows the Candida albicans activity and MLR activity. The six compounds were separated by HPLC using the Chemcosorb (registered trade mark) 5ODS-UH (4.6 mm x 150 mm) eluting with methanol : water = 7:3 to 10:0 at flow rate of 0.7 ml/min at 40°C. The retention times of the compounds are shown in Table 7. The detection was carried out by UV at 280 nm.

Table 7

| Compound | Retention time (min) | $UV\lambda_{max}$ | $LSIMS[M+Na]^+$ |
|---|---|---|---|
| N469-34A | 18.2 | 267,277,288 | 922.5 |
| N469-34B | 12.8 | 267,277,288 | - |
| N460-34C | 13.4 | 267,277,288 | - |
| N369-34D | 14.2 | 267,277,288 | - |
| N469-34E | 15.6 | 267,277,288 | - |
| N469-34F | 19.1 | 267,277,288 | - |
| Rapamycin | 19.4 | 267,277,288 | - |

This invention propose the novel culture, Streptomyces hygroscopicus FERM BP-3688. The macrocyclic lactones which were produced by the culture Streptomyces hygroscopicus (FERM BP-3688), Actinoolanes sp. (FERM BP-3832) or Streptomyces toyocaensis subsp. humicola (ATCC 394871) are useful for an immunosuppressive agent, an antimycotic agent and an anti tumor agent.

## Claims

**1.** A biologically pure culture of Streptomyces hygroscopicus, FERM BP-3688.

**2.** Macrocyclic lactone compounds having immunosuppressive, antimycotic or antitumor activity which are produced by aerobic fermentation of Streptomyces hygroscopicus (FERM BP-3688);or by aerobic fermentation of Actinoplanes sp. (FERM BP-3832) in the presence of the enzyme inhibitor 2-methyl-1,2-di(3-pyridyl)-1-propanone; or by aerobic fermentation of Streptomyces toyocaensis subsp. humicola (ATCC 39471) in the presence of rapamycin.

**3.** Compounds having the following chemical formula:

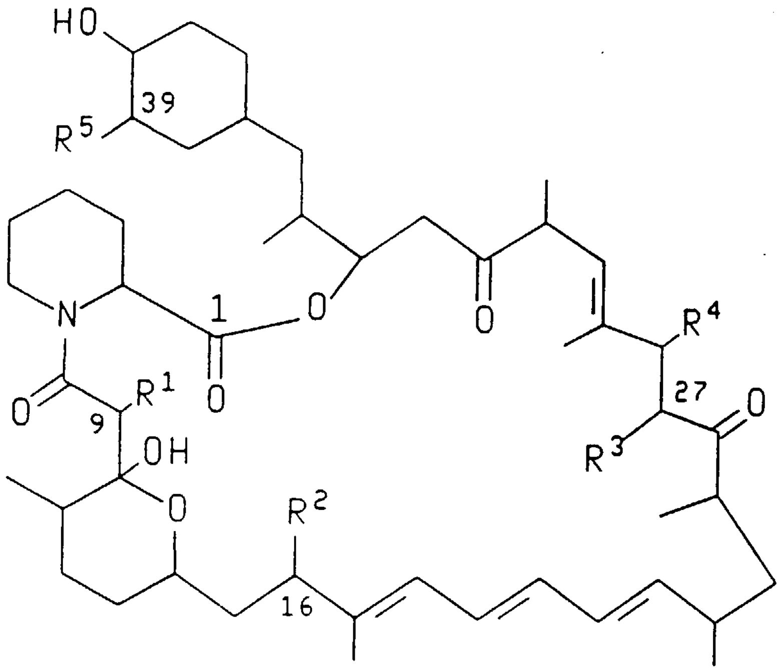

wherein $R^1$ is oxygen, two hydrogens or one hydrogen and one hydroxy;
$R^2$, $R^3$ and $R^5$ are each independently hydrogen, hydroxy or methoxy; and
$R^4$ is hydroxy or hydrogen; provided that when $R^1$ is oxygen, $R^4$ is hydroxy and $R^2$ and $R^5$ are methoxy, $R^3$ is neither hydrogen nor methoxy.

**4.** A compound according to claim 3, in which either:

$R^1$ is oxygen, $R^2$, $R^3$ and $R^4$ are hydroxy and $R^5$ is methoxy;
$R^1$ is oxygen, $R^2$ and $R^4$ are hydroxy and $R^3$ and $R^5$ are methoxy;
$R^1$ is oxygen, $R^2$ is methoxy, $R^3$ is hydrogen and $R^4$ and $R^5$ are hydroxy;
$R^1$ is oxygen, $R^2$ and $R^5$ are methoxy and $R^3$ and $R^4$ are hydroxy;
$R^1$ is oxygen, $R^2$, $R^3$ and $R^5$ are methoxy and $R^4$ is hydrogen;
$R^1$ is two hydrogens, $R^2$, $R^3$ and $R^5$ are methoxy and $R^4$ is hydrogen;
$R^1$ is two hydrogens, $R^2$, $R^3$ and $R^5$ are methoxy and $R^4$ is hydroxy;
$R^1$ is two hydrogens, $R^2$ and $R^5$ are methoxy, $R^3$ is hydrogen and $R^4$ is hydroxy;
$R^1$ is two hydrogens, $R^2$ is methoxy, $R^3$ is hydrogen and $R^4$ and $R^5$ are hydroxy; or
$R^1$ is oxygen, $R^2$ and $R^3$ are methoxy and $R^4$ and $R^5$ are hydroxy.

**5.** A compound according to claim 2 which has characteristic m/z 878.5 $[M+Na]^+$ in LSI mass spectrum.

**6.** An immunosuppressive composition which is characterized by comprising at least one compound according to claim 2 together with an inert carrier.

**7.** An antimycotic composition which is characterized by comprising at least one compound according to claim 2 together with an inert carrier.

**8.** An antitumor composition which is characterized by comprising at least one compound as defined in claim 2 together with an inert carrier.

**9.** A process for the production of the macrocyclic lactone compound, which comprises cultivating Streptomyces hygroscopicus (FERM BP-3688); or by cultivating Actinoplanes sp. (FERM BP-3832) in the presence of the enzyme inhibitor 2-methyl-1,2-di(3-pyridyl)-1-propanone; or by cultivating Streptomyces toyocaensis subsp. humicola

(ATCC 39471), in the presence of rapamycin;

in an aqueous nutrient medium containing assimilable sources of carbon and nitrogen, under aerobic conditions; and wherein the macrocyclic lactone compound has the formula:

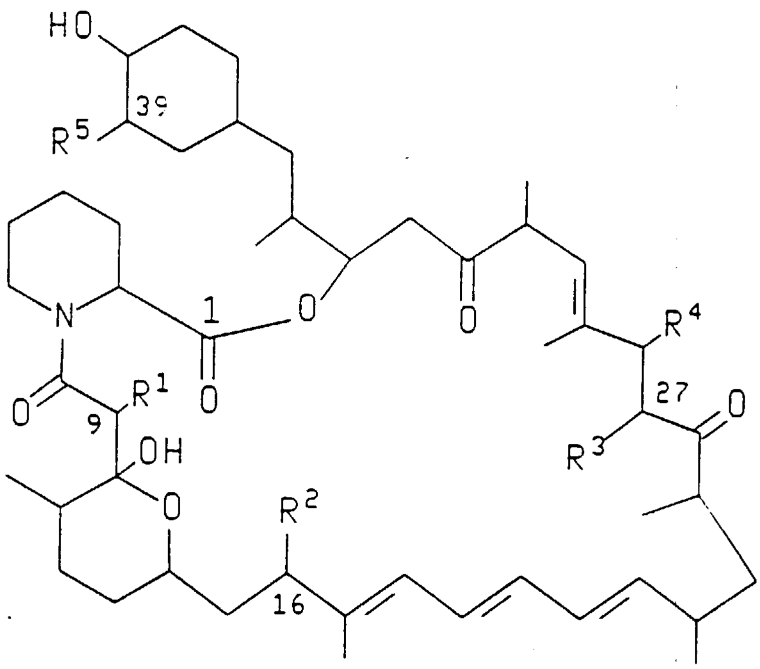

wherein $R^1$ is oxygen, two hydrogens or one hydrogen and one hydroxy;
$R^2$, $R^3$ and $R^5$ are each independently hydrogen, hydroxy or methoxy; and
$R^4$ is hydroxy or hydrogen; provided that when $R^1$ is oxygen, $R^4$ is hydroxy, and both $R^2$ and $R^5$ are methoxy ; then $R^3$ is neither hydrogen nor methoxy.

**Patentansprüche**

1. Biologisch reine Kultur von Streptomyces hygroscopicus, FERM BP-3688.

2. Makrocyclische Lactonverbindungen mit immunosuppressiver, antimycotischer oder Antitumorwirkung, hergestellt durch aerobe Fermentation von Streptomyces hygroscopicus (FERN BP-3688); oder durch aerobe Fermentation von Actinoplanes sp. (FERN BP-3832) in Gegenwart des Enzyminhibitors 2-Methyl-1,2-di(3-pyridyl)-1-propanon; oder durch aerobe Fermentation von Streptomyces toyocaensis subsp. humicola (ATCC 39471) in Gegenwart von Rapamycin.

3. Verbindungen der allgemeinen chemischen Formel:

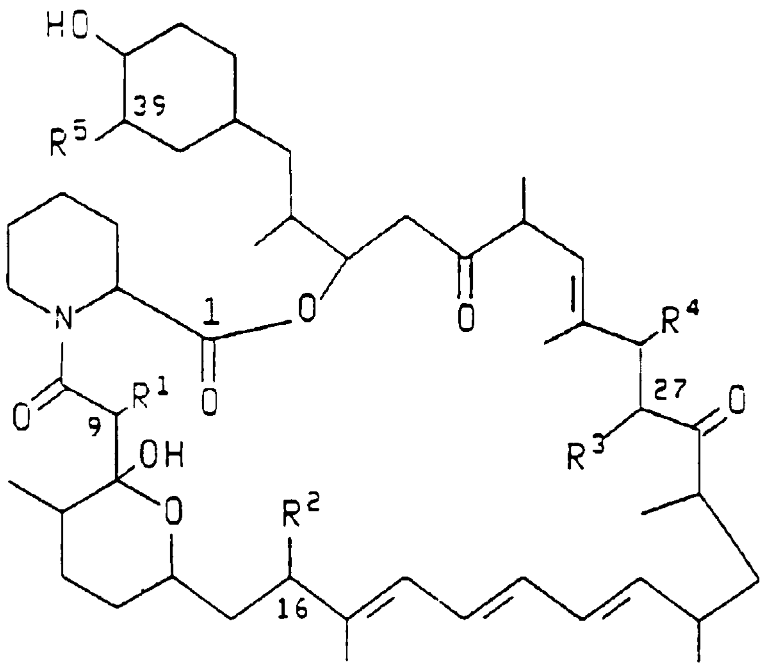

worin $R^1$ Sauerstoff, zwei Wasserstoffatome oder ein Wasserstoffatom und eine Hydroxygruppe bedeutet; $R^2$, $R^3$ und $R^5$ jeweils unabhängig voneinander Wasserstoff, Hydroxy oder Methoxy bedeuten; und $R^4$ Hydroxy oder Wasserstoff bedeutet; mit der Maßgabe, daß wenn $R^1$ Sauerstoff ist, $R^4$ Hydroxy bedeutet und $R^2$ und $R^5$ Methoxy sind, $R^3$ weder Wasserstoff noch Methoxy ist.

**4.** Verbindung nach Anspruch 3, worin entweder:

$R^1$ Sauerstoff bedeutet, $R^2$, $R^3$ und $R^4$ Hydroxy sind und $R^5$ Methoxy ist;
$R^1$ Sauerstoff ist, $R^2$ und $R^4$ Hydroxy sind und $R^3$ und $R^5$ Methoxy sind;
$R^1$ Sauerstoff ist, $R^2$ Methoxy ist, $R^3$ Wasserstoff ist und $R^4$ und $R^5$ Hydroxy sind;
$R^1$ Sauerstoff ist, $R^2$ und $R^5$ Methoxy sind und $R^3$ und $R^4$ Hydroxy sind;
$R^1$ Sauerstoff ist, $R^2$, $R^3$ und $R^5$ Methoxy sind und $R^4$ Wasserstoff ist;
$R^1$ zwei Wasserstoffatome bedeutet, $R^2$, $R^3$ und $R^5$ Methoxy sind und $R^4$ Wasserstoff ist;
$R^1$ zwei Wasserstoffatome bedeutet, $R^2$, $R^3$ und $R^5$ Methoxy sind und $R^4$ Hydroxy ist;
$R^1$ zwei Wasserstoffatome bedeutet, $R^2$ und $R^5$ Methoxy sind, $R^3$ Wasserstoff ist und $R^4$ Hydroxy ist;
$R^1$ zwei Wasserstoffatome bedeutet, $R^2$ Methoxy ist, $R^3$ Wasserstoff ist und $R^4$ und $R^5$ Hydroxy sind; oder
$R^1$ Sauerstoff ist, $R^2$ und $R^3$ Methoxy sind und $R^4$ und $R^5$ Hydroxy sind.

**5.** Verbindung nach Anspruch 2 mit einem charakteristischen m/z-Wert von 878,5 $[M+Na]^+$ im LSI-Massenspektrum.

**6.** Immunosuppressives Mittel, dadurch gekennzeichnet, daß es mindestens eine Verbindung nach Anspruch 2 zusammen mit einem inerten Träger umfaßt.

**7.** Antimycotisches Mittel, dadurch gekennzeichnet, daß es mindestens eine Verbindung nach Anspruch 2 zusammen mit einem inerten Träger umfaßt.

**8.** Antitumormittel, dadurch gekennzeichnet, daß es mindestens eine Verbindung nach Anspruch 2 zusammen mit einem inerten Träger umfaßt.

**9.** Verfahren zur Herstellung der makrocyclischen Lactonverbindung, umfassend Züchten von Streptomyces hygroscopicus (FERM BP-3688); oder Züchten von Actinoplanes sp. (FERM BP-3832) in Gegenwart des Enzyminhibitors 2-Methyl-1,2-di(3-pyridyl)-1-propanon; oder Züchten von Streptomyces toyocaensis subsp. humicola (ATCC 39471) in Gegenwart von Rapamycin;

in einem wässerigen Nährmedium, das assimilierbare Quellen für Kohlenstoff und Stickstoff enthält, unter aeroben Bedingungen; und wobei die makrocyclische Lactonverbindung die Formel aufweist:

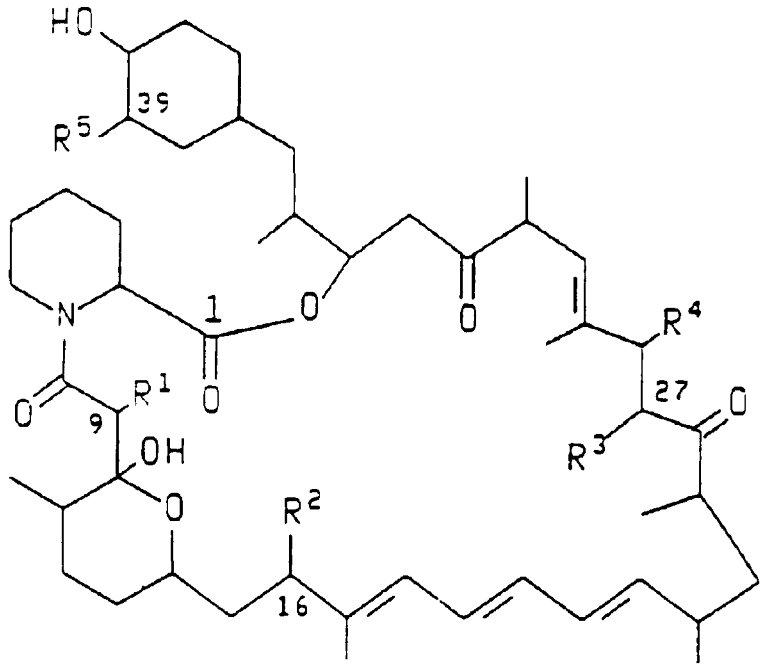

worin $R^1$ Sauerstoff, zwei Wasserstoffatome oder ein Wasserstoffatom und eine Hydroxygruppe bedeutet; $R^2$, $R^3$ und $R^5$ jeweils unabhängig voneinander Wasserstoff, Hydroxy oder Methoxy bedeuten; und $R^4$ Hydroxy oder Wasserstoff bedeutet; mit der Maßgabe, daß wenn $R^1$ Sauerstoff ist, $R^4$ Hydroxy bedeutet und $R^2$ und $R^5$ Methoxy sind, $R^3$ weder Wasserstoff noch Methoxy ist.

## Revendications

1. Culture biologiquement pure de Streptomyces hygroscopicus, FERM BP-3688.

2. Composés lactones macrocycliques présentant une activité immuno-suppressive, antimycotique ou antitumorale qui sont obtenus par fermentation aérobie de Streptomyces hygroscopicus (FERM BP-3688); ou par fermentation aérobie d'Actinoplanes sp. (FERM BP-3832) en présence de l'inhibiteur d'enzyme 2-méthyl-1,2-di(3-pyridyl)-1-propanone; ou par fermentation aérobie de Streptomyces toyocaensis subsp. humicola (ATCC 39471) en présence de rapamycine.

3. Composés ayant la formule chimique suivante :

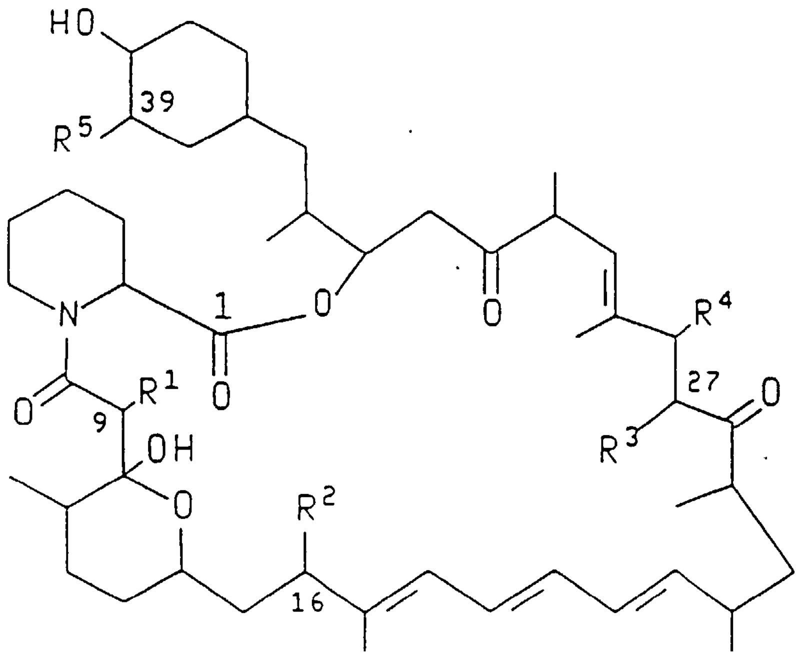

dans laquelle $R^1$ représente l'oxygène, deux atomes d'hydrogène, ou un atome d'hydrogène et un groupe hydroxy;
$R^2$, $R^3$ et $R^5$ représentent chacun indépendamment l'hydrogène, un groupe hydroxy ou méthoxy; et
$R^4$ représente l'hydrogène ou un groupe hydroxy; à condition que lorsque $R^1$ représente l'oxygène, $R^4$ représente un groupe hydroxy et $R^2$ et $R^5$ représentent le groupe méthoxy, $R^3$ ne représente ni l'hydrogène ni le groupe méthoxy.

**4.** Composé selon la revendication 3, dans lequel soit :

$R^1$ représente l'oxygène, $R^2$, $R^3$ et $R^4$ représentent le groupe hydroxy et $R^5$ représente le groupe méthoxy;
$R^1$ représente l'oxygène, $R^2$ et $R^4$ représentent le groupe hydroxy et $R^3$ et $R^5$ représentent le groupe méthoxy;
$R^1$ représente l'oxygène, $R^2$ représente le groupe méthoxy, $R^3$ représente l'hydrogène et $R^4$ et $R^5$ représentent le groupe hydroxy;
$R^1$ représente l'oxygène, $R^2$ et $R^5$ représentent le groupe méthoxy et $R^3$ et $R^4$ représentent le groupe hydroxy;
$R^1$ représente l'oxygène, $R^2$, $R^3$ et $R^5$ représentent le groupe méthoxy et $R^4$ représente l'hydrogène;
$R^1$ représente deux atomes d'hydrogène, $R^2$, $R^3$ et $R^5$ représentent le groupe méthoxy et $R^4$ représente l'hydrogène;
$R^1$ représente deux atomes d'hydrogène, $R^2$, $R^3$ et $R^5$ représentent le groupe méthoxy et $R^4$ représente le groupe hydroxy;
$R^1$ représente deux atomes d'hydrogène, $R^2$ et $R^5$ représentent le groupe méthoxy, $R^3$ représente l'hydrogène et $R^4$ représente le groupe hydroxy;
$R^1$ représente deux atomes d'hydrogène, $R^2$ représente le groupe méthoxy, $R^3$ représente l'hydrogène et $R^4$ et $R^5$ représentent le groupe hydroxy; ou $R^1$ représente l'oxygène,
$R^2$ et $R^3$ représentent le groupe méthoxy et $R^4$ et $R^5$ représentent le groupe hydroxy.

**5.** Composé selon la revendication 2 qui présente le rapport caractéristique m/z 878,5 $[M+Na]^+$ dans son spectre de masse LSI.

**6.** Composition immuno-suppressive qui est caractérisée en ce qu'elle comprend au moins un composé selon la revendication 2 avec un vecteur inerte.

**7.** Composition antimycotique qui est caractérisée en ce qu'elle comprend au moins un composé selon la revendication 2 avec un vecteur inerte.

**8.** Composition antitumorale qui est caractérisée en ce qu'elle comprend au moins un composé selon la revendication

2 avec un vecteur inerte.

9. Procédé de production du composé lactone macrocyclique, qui comprend la cultivation de Streptomyces hygroscopicus (FERM BP-3688); ou la cultivation d'Actinoplanes sp. (FERM BP-3832) en présence de l'inhibiteur d'enzyme 2-méthyl-1,2-di(3-pyridyl)-1-propanone; ou la cultivation de Streptomyces toyocaensis subsp. humicola (ATCC 39471), en présence de rapamycine;

dans un milieu aqueux nutritif contenant des sources assimilables de carbone et d'azote, sous conditions aérobies; et dans lequel le composé lactone macrocyclique a la formule :

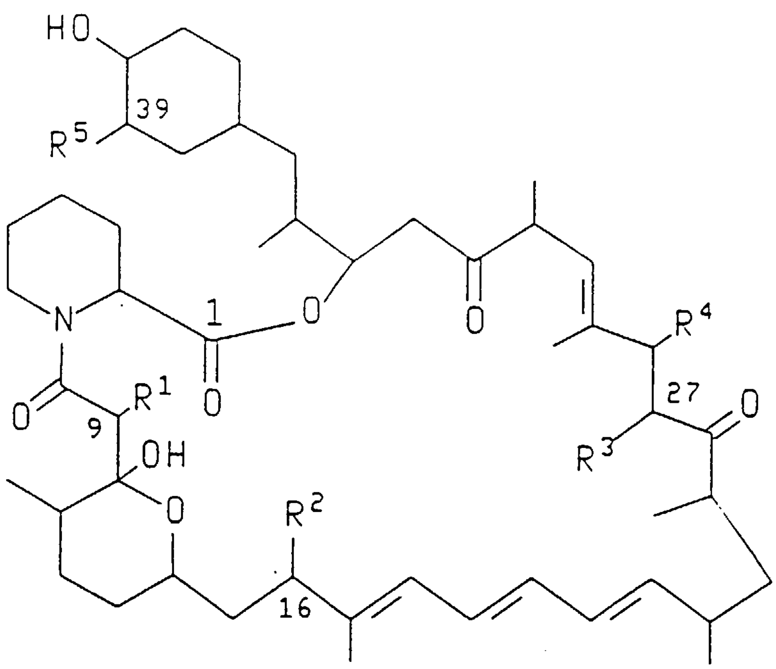

dans laquelle $R^1$ représente l'oxygène, deux atomes d'hydrogène ou un atome d'hydrogène et un groupe hydroxy;
$R^2$, $R^3$ et $R^5$ représentent chacun indépendamment l'hydrogène, un groupe hydroxy ou méthoxy; et
$R^4$ représente l'hydrogène ou un groupe hydroxy; à condition que lorsque $R^1$ représente l'oxygène, $R^4$ représente un groupe hydroxy et $R^2$ et $R^5$ représentent tous deux le groupe méthoxy; alors $R^3$ ne représente ni l'hydrogène ni le groupe méthoxy.